(19) Europäisches Patentamt
European Patent Office
Office européen des brevets

(11) EP 1 016 405 A2

(12) # EUROPEAN PATENT APPLICATION

(43) Date of publication:
**05.07.2000 Bulletin 2000/27**

(21) Application number: **99204250.7**

(22) Date of filing: **23.05.1994**

(51) Int Cl.[7]: **A61K 31/04**, A61K 31/06, A61K 31/136, A61K 31/165, A61K 31/185

(84) Designated Contracting States:
**AT BE CH DE DK ES FR GB GR IE IT LI LU MC NL PT SE**

(30) Priority: **21.05.1993 GB 9310520**
**17.03.1994 GB 9405292**

(62) Document number(s) of the earlier application(s) in accordance with Art. 76 EPC:
**94915671.5 / 0 700 287**

(71) Applicant: **Radopath Pharmaceuticals International Limited**
**St Peter Port Guernsey**
**Channel Islands GY1 6AX (GB)**

(72) Inventor: **Ayuko, Washington Odur**
**Kingstanding, Birmingham B44 9NY (GB)**

(74) Representative: **Matthews, Derek Peter**
**Frank B. Dehn & Co.,**
**European Patent Attorneys,**
**179 Queen Victoria Street**
**London EC4V 4EL (GB)**

Remarks:
This application was filed on 10 - 12 - 1999 as a divisional application to the application mentioned under INID code 62.

(54) **Arylating medicaments**

(57) Various arylating agents having activity in the treatment of cancer and viral infection are disclosed. The active compounds include an aromatic ring having at least one labile leaving group and at least one electrophilic group. Preferred active compounds include chlorobenzenesulphonic acids and optionally halogenated nitrobenzenc compounds. In an anti-viral context, the active compounds have efficacy against HIV infections.

EP 1 016 405 A2

## Description

The present invention relates to arylating agents, in particular phenylating agents, which are suitable as therapeutic compounds, especially in the treatment of cancer and disease caused by viral infection.

In its broadest sense, the invention relates to arylating agents for use in the treatment of neoplasm or of viral infection such as by HIV. The arylating agent will in particular be a compound having an aryl group whose aromatic ring is preferably carbocyclic and has in any event at least one labile substituent and at least one electrophilic substituent. The carbocyclic or other aromatic ring is preferably monocyclic and in any event the aromatic ring is conveniently one which bears one or more carboxylic acid or sulphonic acid moieties together with one or more nitro and/or amino groups and/or one or more halogen substituents. The substituents preferably do not include more than two nitro substituents. A combination of halogen (eg. chloro) and nitro substituents, especially in the context of a monocyclic arylating agent comprised of a ring carrying a carboxylic acid substituent, is a particularly efficacious structure. One example of such a structure is one based on a combination of mono-nitro- and mono-chloro- substitution (eg. 2-chloro-5-nitro benzoic acid and 2-chloro-4-nitro benzoic acid).

According to the invention there is provided a compound for use in the treatment of cancer or disease caused by viral infection, in particular AIDS, which compound comprises an aromatic ring structure having at least one labile leaving group substituent and at least one electrophilic group substituent provided that where there are two ortho nitro groups and a para sulphonic group or three symmetrical nitro groups and the labile group at position one is a group as defined in International Specification No. WO91/15200, use is at a concentration of more than 1 x $10^{-3}$ moles/litre.

Generally speaking the compound of the invention may be of the general formula:

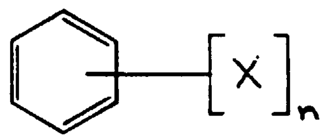

(I)

wherein n is an integer and is at least 2 and each X is the same or different and is a labile group or an electrophilic group, provided that when there are at least two groups X which are other than nitro at least one is a labile group and at least one is an electrophilic group.

Moreover, since treatment is sought by what is believed to be an arylating mechanism use is typically at relatively high concentrations and consequently doses. Generally, such concentrations for use of the compounds of the invention will be at least about 1 x $10^{-2}$ moles/litre, which in dosage terms is generally at least about 5 mg/kg

In selecting the substituent groupings for a compound according to the invention an essential feature is the provision within any particular aromatic ring context of at least one labile group substituent and at least one electrophilic group substituent. Moreover, a group which may be classified as labile within one particular ring context may be classifiable as electrophilic within another alternative ring context. Furthermore, where there are at least two nitro substituents the labile group substituent may be a ring hydrogen.

That having been understood preferred substituent groups may be defined as those wherein at least one X is selected from each of the following groups, namely:

| | |
|---|---|
| electrophilic groups - | $SO_3H$, $SO_3M$ (where M is a metal e.g. potassium), halogen and $NO_2$. |
| labile groups - | halogen, $SO_3H$, $SO_3M$ (where M is a metal), $NH_2$, substituted $NH_2$ e.g. $NHR_1$, $NR_1R_2$ (where $R_1$, and $R_2$ are the same or different and are each alkyl, alkyloxy or hydroxyalkyl), COOH, $CONH_2$, substituted $CONH_2$ e.g. $CONHR_1$, $CONR_1R_2$ (where $R_1$ and $R_2$ are as defined above) and $COOR_3$ (where $R_3$ is a metal or alkyl). |

Thus, as general examples of compounds of the invention there may be mentioned the following, namely: chlorodinitrobenzenesulphonic acids chlorobenzenesulphonic acids dichlorobenzenesulphonic acids aminodinitrobenzenesulphonic acids nitromethylbenzenesulphonic acids glutathionyldinitrobenzenesulphonic acids nitrochlorobenzenesulphonic acids dinitrobenzenesulphonic acids dinitrochlorobenzenes dinitrofluorobenzenes dichlorodinitrobenzenes
trinitrophenols e.g. picric acid trinitroanilines trinitrochlorobenzenes trinitrobenzenesulphonic acids chlorodinitrobenzoic acids dichlorobenzoic acids dinitrobenzoic acids nitrochloroanisoles aminodinitrobenzamides dinitroanilines dinitrochloroanilines chloronitroanilines dinitrofluoroanilines

The above compounds may typically be summarised by compounds of the general formula:

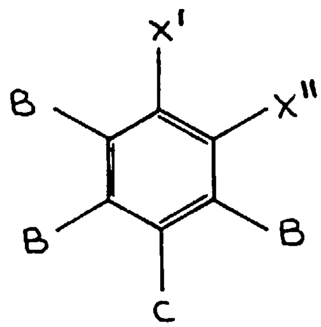

(II)

wherein X' is $SO_3H$, $SO_3M$ (where M is a metal), halogen e.g. chloro, fluoro etc., COQ (where Q is hydroxy, amino or substituted amino, or the group $OR_3$ in which $R_3$ is a metal or alkyl), $NH_2$, substituted $NH_2$, $NO_2$ or OH,

X" is hydrogen, halogen, glutathione or nitro,
each B is the same or different and is hydrogen,
halogen or nitro and

C is hydrogen, nitro, amino (including substituted amino), halogen, alkyl or glutathione.

In such compounds the following are preferred features:

X' is $SO_3H$, $SO_3H$ (where M is a metal), halogen e.g. chloro, fluoro etc., amino, nitro or COOH, and

C is hydrogen, alkyl e.g. methyl, amino or nitro.

The compounds which exhibit anti-cancer and anti-viral effects according to the invention may be sub-divided into a number of preferred groupings, for example, as follows:

(i) A compound of the general formula:

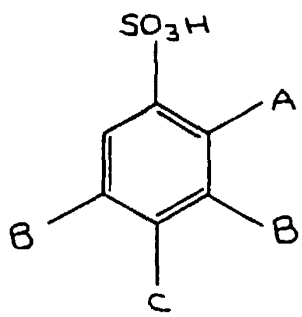

(III)

wherein

A is hydrogen, halogen e.g. chloro, fluoro etc., or glutathione,

B is hydrogen, nitro or halogen e.g. chloro etc.,

C is hydrogen, nitro, amino (including substituted amino), halogen, alkyl or glutathione, and

D is hydrogen, halogen or nitro.

The above compounds of formula III are preferred because it is believed that the sulphonic grouping can

contribute an emulsifying effect which is useful because it increases the solubility of the compounds, which in turn gives better bioavailability in cellular terms.

Amongst the above compounds of formula III, those more preferred are:

4-chloro-3,5-dinitrobenzenesulphonic acid
4-chlorobenzenesulphonic acid
2,5-dichlorobenzenesulphonic acid
4-amino-3,5-dinitrobenzenesulphonic acid
3-nitro-4-methylbenzenesulphonic acid
2-chloro-3,5-dinitrobenzenesulphonic acid
2-glutathionyl-3,5-dinitrobenzenesulphonic acid
4-glutathionyl-3,5-dinitrobenzenesulphonic acid
3-nitro-4-methylbenzenesulphonic acid
3-nitro-4-chlorobenzenesulphonic acid
2,4-dinitrobenzenesulphonic acid.

Especially preferred are:

4-chloro-3,5-dinitrobenzenesulphonic acid
4-chlorobenzenesulphonic acid
2,5-dichlorobenzenesulphonic acid
4-amino-3,5-dinitrobenzenesulphonic acid
3-nitro-4-methylbenzenesulphonic acid
2-chloro-3,5-dinitrobenzenesulphonic acid

(ii) A compound of the general formula:

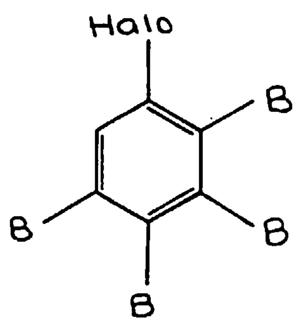

(IV)

wherein halo is halogen e.g. chlorine, fluorine etc., and each B is the same or different and is as defined above.

Amongst the above compounds of formula IV, those more preferred are:

1-chloro-2,4-dinitrobenzene
1-chloro-3,4-dinitrobenzene
1-fluoro-2,4-dinitrobenzene
1,2-chloro-4,5-dinitrobenzene
1,3-chloro-4,5-dinitrobenzene.

Especially preferred are:

1,3-chloro-4,5-dinitrobenzene
1-chloro-2,4-dinitrobenzene
1-fluoro-2,4-dinitrobenzene

(iii) A compound of the general formula:

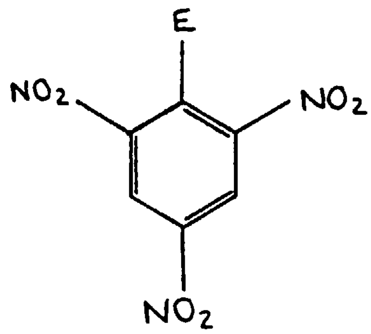

(V)

wherein E is $SO_3H$, $SO_3M$ (where M is a metal e.g. potassium), $NH_2$ or substituted $NH_2$, halogen or hydroxy. Amongst compounds of formula V, those more preferred are:

2,4,6-trinitrophenol (picric acid),
2,4,6-trinitroaniline,
2,4,6-trinitrochlorobenzene.
2,4,6-trinitrobenzenesulphonic acid.

Of the above preferred compounds the first and third are especially preferred.

(iv) A compound of the general formula:

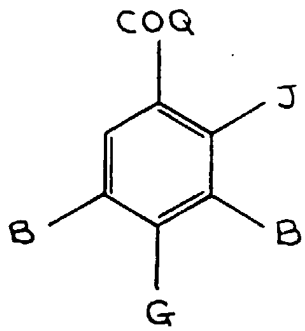

(VI)

wherein each B is the same or different and is as defined above,

G is as defined above for group C except for alkyl and glutathione,

J is hydrogen or halogen, and

Q is hydroxy, amino or substituted amino, or the group $OR_3$ in which $R_3$ is a metal or alkyl.

Amongst compounds of formula VI, those more preferred are:

2,4-chloro-3,5-dinitrobenzoic acid
4-chloro-3,5-dinitrobenzoic acid
2,5-dichlorobenzoic acid
2,4-dinitrobenzoic acid
3,5-dinitrobenzoic acid
3-nitro-4-chloroanisole
4-amino-3,5 dinitrobenzamide

Of the above preferred compounds, all but the last three are especially preferred.

(v) A compound of the general formula:

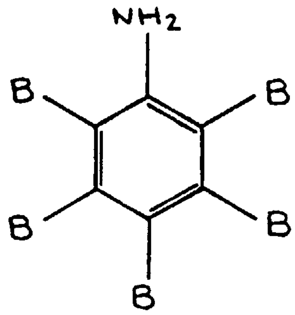

(VII)

wherein each B is the same or different and is as defined above, together with amino substituted derivatives thereof.

Amongst compounds of formula VII, those more preferred are:

2,6-dinitroaniline
2,4-dinitroaniline
3,5-dinitroaniline
2,4-dinitro-6-chloroaniline
2,6-dinitro-4-chloroaniline
2-chloro-4-nitro aniline
2,4-dinitro-5-fluoroaniline

Especially preferred is:
2,6-dinitroaniline

As mentioned above, where there are at least two nitro substituents a ring hydrogen may provide a labile group. Within that context there may be mentioned:

(vi) A compound of the general formula:

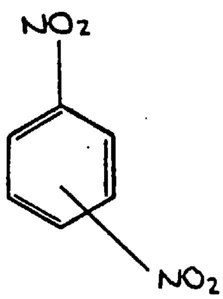

(VIII)

that is to say:

1,2-dinitrobenzene
1,3-dinitrobenzene
1,4-dinitrobenzene

The compounds of the invention may be prepared by known process techniques for preparing benzene substituted compounds. Such techniques are described in various standard texts, for example, "Organic Syntheses" 1963 Collective Volume 4, pages 364 to 366, by Harry P. Schultz and published by John Wiley and Sons Inc.

The compounds of the invention may be formulated for use as pharmaceutical compositions (eg for iv, ip, oral or sc administration) comprising at least one active compound and a diluent or carrier. Thus, the invention includes a pharmaceutical composition, which composition comprises a compound according to the invention and a pharmaceutically-acceptable diluent or carrier (eg aqueous).

Such a composition may be in bulk form or, more preferably, unit dosage form. Thus, for example, the composition may be formulated as a tablet, capsule, powder, solution or suspension. Soft gel capsules may be especially

convenient. The composition may be a liposomal formulation or administered in a slow sustained release delivery system.

Compositions in accordance with the invention may be prepared using the active compounds defined herein in accordance with conventional pharmaceutical practice. The diluents, excipients or carriers etc. which may be used are well known in the formulation art and the form chosen for any particular regimen will depend on the given context and the physician's choice.

Thus, for example, as illustrated below the compounds of the invention may be administered in solution in sterile deionised water. Also, if necessary, solution may be facilitated using dimethyl sulphoxide (DMSO) or alternatively an alcohol, a glycol or a vegetable oil. The compounds are most favourably administered in corn oil or as a solution in DMSO/sterile water.

The invention further includes within the above use context the use of a compound as defined herein in the preparation of a medicament for the prophylaxis or therapy of cancer or viral infection, eg to reduce or eliminate cancerous growth.

In using a compound of the invention dosage guidance can be taken from animal studies such as that described below. In such studies doses of from about 50 mg/kg typically up to about 200 mg/kg and even up to about 400 mg/kg and beyond have proved effective. Thus it is to be expected that a typical dosage for humans will be from about 5 mg/kg typically to about 20 mg/kg and perhaps generally to about 40 mg/kg or higher. The concentration and dose are to be sufficient to bring an arylating mechanism into play.

As can be seen from the especially preferred compounds listed above, those compounds of the invention which are most efficacious are in believed descending order of activity as follows, namely:

4-chloro-3,5-dinitrobenzenesulphonic acid
4-chlorobenzenesulphonic acid
1,5-chloro-2,3-dinitrobenzene
2,4,6-trinitrophenol (picric acid)
2,4-chloro-3,5-dinitrobenzoic acid
2,5-dichlorobenzenesulphonic acid
4-amino-3,5-dinitrobenzenesulphonic acid
3-nitro-4-methylbenzenesulphonic acid
4-chloro-3,5-dinitrobenzoic acid
2,6-dinitroaniline
2,4-dinitrochlorobenzene
2,4-dinitrofluorobenzene
2,4,6-trinitrochlorobenzene
2,5-dichlorobenzoic acid
2-chloro-3,5-dinitrobenzenesulphonic acid
2,4-dinitrobenzoic acid

Especially preferred compounds are those wherein at least one X is selected from:

| | |
|---|---|
| labile substituent group(s) - | 1 or 2 halogen groups and/or $NH_2$ or substituted $NH_2$ and/or COOH or substituted COOH and/or alkyl and/or $SO_3H/SO_3M$ |
| electrophilic substituent group(s) - | 1 or 2 nitro groups and/or $SO_3H/SO_3M$ and/or 1 or 2 halogen groups |

Moreover, while the compounds of the invention can be used within the dosage regimen exemplified above, where there are three symmetrical nitro substituents or the active agent is otherwise as disclosed in International Specification No WO 91/15200, as indicated above, the concentration of active agent in any formulation must be more than 1 X $10^{-3}$ moles/litre and preferably at least 1 X $10^{-2}$ moles/litre.

As shown by the results reported in Table 8 below, 2-chloro-5-nitrobenzoic acid shows consideration anti-tumour activity in vivo. This could not be supported in vitro and it appears some compounds according to the invention require activation in the patient's liver. This and some other compounds may also be immunomodulators.

The following animal study illustrates the remarkable activity of compounds of the invention.

## ANIMAL STUDIES

The purpose of these studies was to evaluate the anti-tumour properties of a group of compounds with structural similarities that may act as arylating agents. Their in vivo anti-tumour responses were assessed against two ascitic tumours, the MAC15A murine colon adenocarcinoma and the P388 murine leukaemia and various solid tumour models.

The MAC15A ascites tumour cells were transplanted into male NMR1 mice by ip inoculation at a cell density of 1 X $10^5$ cells in 200µl buffer (Table 1). The P388 were transplanted ip into male BDF1 mice at cell density of 1 X $10^6$ cells in 200µl buffer (Table 2). The solid tumour models included the MAC13 and MAC16 murine colon adenocarcinomas, the B16 F1 murine melanoma and the M5076 reticulum cell sarcoma.
Treatment commenced 3 days after ip transplant or, in the case of solid tumours such as MAC13 and MAC16, treatment commenced when average tumour volumes reached $40mm^3$.
The animals were located in both cases into groups of 5 to 8 animals.
The animals were sacrificed after 12 days or when tumours ulcerated, tumour volume exceeded $1000mm^3$ or loss of body weight exceeded 50%.
Except where otherwise stated, the compounds used were dissolved in DMSO and diluted in sterile distilled water, at appropriate concentrations before administration in a solvent volume of 200 µl. Anti-tumour responses were obtained by comparing the median survival times or tumour growth inhibition against solvent controls. The results obtained are as shown in Tables 1 to 8 below.
*Preparation* of dosage solutions is exemplified as follows:-

| | |
|---|---|
| Subjects: | No : 10 animals<br>Weight: 22g |
| Dosage: | 50mg/kg body weight per animal per day thus 1.1mg per mouse per day |
| Total Mass Dosage: | 55mg active ingredient (referred to 5 day treatment regime) |
| Total Formulation: | 10ml solvent plus 55mg for division into 50 doses of 1.1mg dissolved in 200µl solvent |

*T/C%* is determined as follows:-

| Animal Survival | Test | Control |
|---|---|---|
| | T days | C days |

$$T/C\% = \frac{T}{C} X 100$$

*Example*

| Animal Survival | Test | Control |
|---|---|---|
| | 443 days | 100 days |

$$T/C\% = \frac{443}{100} X 100 = 443$$

A figure of 158 or above indicates performance justifying clinical trial.

*Conclusions*

The effect of a group of primarily halogenated arylating compounds on the growth rate of a number of experimental tumours has been evaluated *in vivo* and the following findings were noted:

1. Structure-activity relationships against the MAC15A murine colon adenocarcinoma, in the female NMRI mice showed maximal activity on a split-dose schedule and when the halogen was maximally activated for nucleophilic attack.

2. The most active compound was 4-chlorobenzenesulphonic acid (T/C% 443) administered at 100 mg/kg body weight in a daily schedule of 5 days.

3. Against the M5076 reticulum cell sarcoma, 2,4-dichloro-3,5-dinitrobenzoic acid showed activity on a split-dose schedule down to 25 mg/kg body weight by both ip and sc routes. Both the amide and the methyl ester showed 10-fold increase in toxicity and were without antitumour activity. The acid also effectively inhibited growth of B16 murine melanoma and the MAC16 murine colon adenocarcinoma.

It is concluded that this group of compounds show a wide spectrum of activity against murine models.

TABLE 1

*Anti-tumour activity against MAC15A (murine adenocarcinoma colon). Structure-Activity relationship. 5 animals per group. Dose 100 mg $kg^{-1}$ ip per day.*

| Compound | Schedule (days) | T/C%[a] |
|---|---|---|
| 4-chlorobenzenesulfonic acid | 1,2,3,4,5 | 443 |
| 4-chloro-3,5-dinitrobenzenesulfonic acid | 1,2,3,4,5 | 414 |
| 1,5-dichloro-2,3-dinitrobenzene | 1,2,3,4,5 | 386 |
| 2,4,6-trinitrophenol | 1,2,3 | 300 |
| 4-amino-3,5-dinitrobenzenesulfonic acid | 1,2,3,4,5 | 286 |
| 4-chloro-3,5-dinitrobenzoic acid | 1,2,3,4,5 | 271 |
| 2,4-dichloro-3,5-dinitrobenzoic acid | 1,2 | 243 |
| 2-glutathionyl-3,5-dinitrobenzenesulfonic acid | 1,2,3,4,5 | 242 |
| 3-nitro-4-methylbenzenesulfonic acid | 1,2,3,4,5 | 229 |
| 2,6-dinitroaniline | 1,2,3,4,5 | 214 |
| 2,5-dichlorobenzenesulfonic acid | 1,2,3,4,5 | 212 |
| 1,4-dinitrobenzene | 1,2 | 200 |
| 1-chloro-3,4-dinitrobenzene | 1,2,3,4,5 | 200 |
| 1-chloro-2,4-dinitrobenzene | 1,2,3,4,5 | 188 |
| 2,4,6-trinitrobenzenesulfonic acid | 1,2,3,4,5 | 188 |
| 2-chloro-4-nitroaniline | 1,2,3,4,5 | 171 |
| 2,5-dichlorobenzoic acid | 1,2,3,4,5 | 171 |
| 2,4-dinitrobenzenesulfonic acid | 1,2,3,4,5 | 171 |
| 1,2-dichloro-4,5-dinitrobenzene | 1,2,3,4,5 | 171 |
| 4-chloro-3-nitrobenzenesulfonic acid | 1,2,3,4,5 | 140 |
| 2-chloro-3,5-dinitrobenzenesulfonic acid | 1,2,3,4,5 | 137 |
| 1-chloro-2,4,6-trinitrobenzene | 1,2,3 | 113 |
| 4-glutathionyl-3,5-dinitrobenzene | 1,2,3,4 | 113 |
| 2,4-dinitroaniline | 1,2 | 100 |
| 2,4-dinitrobenzoic acid | 1,2,3,4,5 | 100 |
| 3,5-dinitrobenzoic acid | 1,2,3,4,5 | 100 |
| 4-amino-3,5-dinitrobenzamide | 1 | 100 |
| 4-chloro-3-nitroanisole | 1,2,3,4,5 | 100 |
| 4-chloro-2,6-dinitroaniline | 1,2,3,4,5 | 87 |
| 6-chloro-2,4-dinitroaniline | 1,2,3,4,5 | 87 |
| 1-fluoro-2,4-dinitroaniline | 1 | 75 |
| 1-flouro-2,4-dinitrobenzene | 1 | 62.5[b] |

a=median, T-test group, C-solvent control;
b-toxic death

TABLE 2

| Anti-tumour activity against P388 (murine leukaemia). Eight animals per group. IP treatment on day 1 to 5. Dosage is per day. | | |
|---|---|---|
| Compound | Dose | TC%[a] |
| 4-chloro-3,5-dinitrobenzene-sulphonic acid | 100mg $kg^{-1}$ | 203 |
| 4-chloro-3,5-dinitrobenzene-sulphonic acid | 50 mg $kg^{-1}$ | 259 |

a=mean, T=test group, C=solvent control.

TABLE 3

| Anti-tumour activity against P388 (murine leukaemia) treated ip with 4-chloro-3,5-dinitrobenzenesulfonic acid (CDNSA). 8 animals per group. Dosage is per day. | | | |
|---|---|---|---|
| Compound | Dose (mg/kg) | Schedule (days) | T/C%[a] |
| CDNSA | 100 | 1,2,3,4,5 | 2 2 5 |
| | 75 | 1,2,3,4,5 | 3 0 0 |

a=mean, T-test group, C-solvent control

## TABLE 4

*Anti-tumour activity against M5076-reticulum cell sarcoma 16 days after im transplant. 7 animals per group. Drugs dissolved in corn oil. Dosage is per day.*

| Compound | Dose (mg/kg) | Route | Schedule (days) | % Tumour Weight Inhibition |
|---|---|---|---|---|
| 2,4 BA | 75[a] | ip | 1,4,6,9 | 79,88[b] |
| | 50 | ip | 1,4,6,9 | 57 |
| | 25 | ip | 1,2,4,6,9 | 75 |
| | 75 | sc | 1,4,5,7,9 | 66 |
| | 50 | sc | 1,2,4,5,6,7,9 | 76 |
| | 25 | sc | 1,2,4,5,6,7,9 | 63 |
| 2,4 BZ | 2.5[a] | ip | 1,2,3,4,5,6,7,8,9 | 51 |
| | 1.25 | ip | 1,2,3,4,5,6,7,8,9 | 34 |
| 2,4 BM | 1.0[a] | ip | 1,2,3,4,5,6,7,8,9 | 41 |
| | 0.5 | ip | 1,2,3,4,5,6,7,8,9 | 39 |
| | 0.25 | ip | 1,2,3,4,5,6,7,8,9 | 42 |

a = Maximum tolerated dose
b = two independent experiments; 4 animals had no tumour in the second experiment

2,4 BA = 2,4-dichloro-3,5-dinitrobenzoic acid
2,4 BZ = 2,4-dichloro-3,5-dinitrobenzamide
2,4 BM = 2,4-dichloro-3,5-dinitrobenzoic acid methyl ester

% Tumour Weight Inhibition:-

| *Treated* | *Control* | |
|---|---|---|
| Agm | Bgm | Tumour weight |

$$\% \text{ inhibition} = \frac{B - A}{B} \times 100$$

TABLE 5

| Anti-tumour activity against B16F1-murine melanoma 12 days after sc transplant. 6 animals per group. Drugs dissolved in corn oil. Dosage is per day. | | | | |
|---|---|---|---|---|
| Compound | Dose (mg/kg) | Route | Schedule (days) | % Tumour Weight Inhibition |
| 2,4 BA | 75[a] | ip | 1,5 | 71,81[b] |
| | 50 | ip | 1,5 | 45,56[b] |
| | 25 | ip | 1,5 | 13 |
| | 75 | sc | 1,3,5 | 30 |
| | 50 | sc | 1,3,5 | 9 |
| | 25 | sc | 1,3,5 | 22 |
| 2,4 BZ | 2.5[a] | ip | 1,2 | 39 |
| | 1.25 | ip | 1,2 | 17 |
| 4 BA | 100 | ip | 1,5 | 39 |
| | 75 | ip | 1,5 | 41 |
| | 50 | ip | 1,5 | 10 |
| 4 BZ | 5[a] | ip | 1,3,5 | 18 |
| | 2.5 | ip | 1,3,5 | 18 |
| | 1.25 | ip | 1,3,5 | 27 |
| 4BM | 2.5[a] | ip | 1,3 | 67 |
| | 1.25 | ip | 1,2,3 | 43 |

a = Maximum tolerated dose
b = Two independent experiments
2,4 BA = 2,4-dichloro-3,5-dinitrobenzoic acid
2,4 BZ = 2,4-dichloro-3,5-dinitrobenzamide
4 BA = 4-chloro-3,5-dinitrobenzoic acid
4 BZ = 4-chloro-3,5-dinitrobenzamide
4 BM = 4-chloro-3,5-dinitrobenzoic acid methyl ester

TABLE 6

| Anti-tumour activity against MAC13 murine colon adenocarcinoma 12 days after im transplant. Drugs dissolved in corn oil. Dosage is per day. | | | | |
|---|---|---|---|---|
| Compound | Dose (mg/kg) | Route | Schedule (days) | % Tumour Weight Inhibition |
| 2,4 BA | 75[a] | ip | 1,4,5 | 45 |
| 2,4 BA | 50 | ip | 1,2,3,4,5,6,7,8,9 | 39 |
| 2,4 BA | graph[3] | ip | graph[3] | graph[3] |
| 2,4 BZ | 2.5[a] | ip | 1,2,3,4,5,6,7,8,9 | 51 |
| 2,4 BZ | 1.25 | ip | 1,2,3,4,5,6,7,8,9 | 17 |
| 2 BA | graph[4] | ip | graph[4] | graph[4] |

a = maximum tolerated dose
2,4 BA = 2,4-dichloro-3,5-dinitrobenzoic acid[3]
2,4 BZ = 2,4-dichloro-3,5-dinitrobenzamide
2 BA = 2-chloro-5-nitrobenzoic acid
(3: see Figure 3 of the drawings; 4: see Figure 4 of the drawings)

TABLE 7

| Anti-tumour activity against MAC16, murine colon adenocarcinoma sc transplant on day 11 after the beginning of treatment with 2,4-dichloro-3,5-dinitrobenzoic acid (2,4 BA). Drug dissolved in corn oil. The tumour volumes were at least $40mm^3$ at the beginning of the treatment. 6 animals per group. Dosage is per day. | | | | |
|---|---|---|---|---|
| Compound | Dose (mg/kg) | Route | Schedule (days) | % Tumour Weight Inhibition |
| 2,4 BA | 75[a] | ip | 1,2,5,8 | 88 |
| | 50 | ip | 1,2,4,5,8 | 91 |

a = maximum tolerated dose

TABLE 8

| Anti-tumour activity against B16 murine melanoma 12 days after sc transplant on female C57/black mice. 6 animals per group. Dosage is per day and is ip. | | | |
|---|---|---|---|
| Compound | Dose (mg/kg) | Schedule (days) | % Tumour Weight Inhibition |
| 2-chloro-5-nitrobenzoic acid | 700 | 1,2,3,4,5,6 | 62 |

In addition, the following primary assay was used to investigate the anti-viral activity of compounds in accordance with the invention, in particular 4-chloro-3,5-dinitrobenzenesulphonic acid.

Anti-tumour activity and toxicity studies have additionally been completed for the following compounds with broadly satisfactory results:-

C22 2,5-dichloro-4-nitrobenzoic acid
C23 2,4-dichloro-5-nitrobenzoic acid
C24 2,6-dichloro-4-nitrobenzoic acid
C25 2-amino-5-nitrobenzoic acid
C26 2-hydroxy-5-nitrobenzoic acid
C27 3,5-dichloro-4-nitrobenzoic acid

**PRIMARY ASSAY**

(i) *Acute Infection Assay.* High titre virus stocks of the human immunodeficiency virus HIV-$1_{RF}$ were grown in H9 cells with RPMI 1640 (Flow laboratories) supplemented with 10% fetal calf serum, penicillin (100IU/ml). Cell debris was removed by low speed centrifugation, and the supernatant stored at -70°C until required. In a typical assay C8166 T-lymphoblastoid CD4+ cells were incubated with 10xTCID50 HIV-$1_{RF}$ at 37°C for 90 minutes and then washed three times with phosphate buffered saline (PBS). Cell aliquots (2 x $10^5$) were resuspended in 1.5 ml growth medium in 6 ml tubes, and compounds in log dilutions [200μM to 0.2μM] were added immediately. 20 mM stock solutions of each compound were made up in 70% alcohol. The compounds were stored as a powder and made up freshly in distilled water before each experiment or were stored as a 20 mM stock solution in 70% alcohol. The final concentration of alcohol in the tissue culture medium was 1%. The cells were then incubated at 37°C in 5% $CO_2$. At 72 hours post-infection 200 μl of supernatant was taken from each culture and assayed for HIV (Kingchington et al, 1989, Robert et al 1990) using an antigen capture ELISA which recognizes all the core proteins equally (Coulter Electronics, Luton, UK). The following controls were used: supernatants taken from uninfected and infected cells, infected cells treated with AZT (Roche Products UK, Ltd) and ddC (Roche) and R031-8959 (Roche) an inhibitor of HIV proteinase. The $IC_{50}$ activities of 8959, AZT and ddC in infected cells were 1, 10, 20 nM and 200 nM respectively (accompanying Figure 2). The ELISA plates were read with a spectrophotometer. Compounds were tested in duplicate at each concentration, and the data shown is the average of at least two assays. This assay assesses the activity of compounds by measuring their inhibition of HIV core antigen levels.

(ii) *Chronically Infected Cell Assay.* Chronically infected cells (H9rf) were washed three times to remove extracellular virus and incubated with the active compounds (200-0.2 μM) for four days. HIV-1 antigen in the supernatant

was then measured using an ELISA.

To test for compound toxicity uninfected H9 cells were incubated with the compounds for four days. Supernatants were discarded and the cells resuspended in 200µl pg growth medium containing $^{14}C$ protein hydrolysate. After 6 hours the cells were harvested and the $^{14}C$ incorporation measured.

(iii) *Toxicity Assay.* To test for compound toxicity, aliquots of 2 x $10^5$ of uninfected cells were cultured with the compounds in the same dilutions for 72 hours. The cells were then washed with PBSA and resuspended in 200µl of growth medium containing $^{14}C$ protein hydrolysate. After 12 hours the cells were harvested and the $^{14}C$ incorporation measured. Uninfected, untreated cells were used as controls. Toxicity is expressed as inhibition of uptake of $^{14}C$ protein hydrolysate.

The results of these assays for 4-chloro-3,5-dinitrobenzenesulphonic acid are shown in accompanying Figure 1 in which RC stands for Radopath compound C i.e. 4-chloro-3,5-dinitrobenzenesulphonic acid. The results are also summarised in Table 9 below:

TABLE 9

| **Compound** | **$IC_{50}$** | **$CD_{50}$** | **TI** |
|---|---|---|---|
| 4-chloro-3,5-dinitrobenzene-sulphonic acid | 3µM | 80µM | 28.6 |

The $IC_{50}$ is the drug concentration that causes a 50% reduction in HIV core antigen levels as detected by the Coulter P24 antigen assay and is determined by doubling dilutions of supernatant taken from tubes containing untreated acutely infected cells. The $CD_{50}$ is the concentration of drug that causes a 50% inhibition of cells as measured by $^{14}C$ protein hydrolysate uptake. The therapeutic index (TI) is determined by dividing the $CD_{50}$ by the $IC_{50}$.

Further results for other compounds in accordance with the invention are summarised in Table 10 below:

TABLE 10

| **Compound** | **$IC_{50}$** | **$CD_{50}$** | **TI** |
|---|---|---|---|
| 2-chloro-3,5-dinitrobenzenesulphonic acid | 25µm | >200µm | >8 |
| 4-amino-3,5-dinitrobenzenesulphonic acid | 20µm | 100µm | 5 |
| 2,4,6-trinitrophenol | <0.2µm | 95µm | >475 |
| 4-chloro-3,5-dinitrobenzoic acid | 30µm | 70µm | 2.33 |

Initial tests performed approximately contemporaneously indicated 2-chloro-5-nitrobenzoic acid would demonstrate performance at least as efficaceous, if not more so, as any of the compounds whose tests are reported herein.

Following the methodology set forth earlier for performance assay against HIV, more extensive assays were performed as reported in Tables 11 below:

TABLE 11.1

| STRUCTURE-ACTIVITY RELATIONSHIP AGAINST HIV VIRUS | | | |
|---|---|---|---|
| CODE | COMPOUNDS | $^{Ag}IC50$ | $^{Tox}CC50$ |
| GROUP A | | | |
| P1 | picryl chloride | | |
| P2 | picric acid | | |
| P3 | picrylsulfonic acid (sodium salt) | | |
| GROUP B | | | |
| C1 | 2,4-dichloro-3,5-dinitrobenzoic acid | | |
| C2 | 2,4-dichloro-3,5-dinitrobenzamide | | |
| C3 | 2,4-dichloro-3,5-dinitrobenzoic acid methyl ester | | |
| C4 | 4-chloro-3,5-dinitrobenzoic acid | | |
| C5 | 4-chloro-3,5-dinitrobenzamide | | |
| C6 | 4-chloro-3,5-dinitrobenzoic acid methyl ester | | |
| C7 | 2-chloro-3,5-dinitrobenzoic acid | | |

TABLE 11.1 (continued)

| STRUCTURE-ACTIVITY RELATIONSHIP AGAINST HIV VIRUS | | | |
|---|---|---|---|
| CODE | COMPOUNDS | AgIC50 | ToxCC50 |
| GROUP B | | | |
| C8 | 2-chloro-3,5-dinitrobenzoic acid methyl ester | | |
| C9 | 4-chloro-3-nitrobenzoic acid | | |
| C10 | 2-chloro-4-nitrobenzoic acid | | |
| C11 | 3,4-dichlorobenzoic acid | | |
| C12 | 2,5-dichlorobenzoic acid | | |
| C13 | 4-chlorobenzoic acid | | |
| GROUP C | | | |
| S1 | 4-chloro-3,5-dinitrobenzenesulfonic acid | | |
| S2 | 2-chloro-3,5-dinitrobenzenesulfonic acid | | |
| S3 | 4-amino-3,5-dinitrobenzenesulfonic acid | | |
| S4 | 4-chloro-3-nitrobenzenesulfonic acid | | |
| S5 | 4-chlorobenzenesulfonic acid | | |
| S6 | 4-nitrotoilnenesulfonic acid | | |
| S7 | 2,5-dichlorobenzenesulfonic acid | | |
| S8 | 2,4-dinitrobenzenesulfonic acid | | |
| GROUP D | | | |
| E1 | 1-chloro-3,4-dinitrobenzene | | |
| E2 | 1-chloro-2,4-dinitrobenzene | | |
| E3 | 1,2-dichloro-4,5-dinitrobenzene | | |
| E4 | 2,3-dichloronitrobenzene | | |
| E5 | 2,4-dichloronitrobenzene | | |
| E6 | 2,5-dichloronitrobenzene | | |
| E7 | 3,4-dichloronitrobenzene | | |
| E8 | 3,5-dichloronitrobenzene | | |
| E9 | 1,5-dichloro-2,3-dinitrobenzene | | |
| E10 | 1,2,3-trichloro-4-nitrobenzene | | |
| E11 | 1,2,4-trichloro-5-nitrobenzene | | |
| E12 | 2,4,6-trichlorobenzene | | |
| E13 | 2,3,4,6-tetrachloronitrobenzene | | |
| E14 | pentachloronitrobenzene | | |

TABLE 11.2

| P-Compounds | IC50 (Antiviral) | CC50 (Toxicity) | SI (Selectivity Index) |
|---|---|---|---|
| Against HIV-1RF | | | |
| P1 | 0.6 | 7 | 10 |
| | - | 5 | - |
| | 0.4 | - | - |
| **Average** | **0.5** | **6** | **12** |
| P2 | 38 | 67 | 2 |
| P3 | >200 | >200 | - |

TABLE 11.2 (continued)

| P-Compounds | IC50 (Antiviral) | CC50 (Toxicity) | SI (Selectivity Index) |
|---|---|---|---|
| Against HIV-1IIIB | | | |
| P1 | 0.6 | 7 | 11.6 |
| | 1 | 7 | 7 |
| **Average** | **0.8** | **7** | **9** |
| Against chronically infected cells | | | |
| P1 | 0.9 | 7 | 8 |
| | 2 | 12 | 6 |
| **Average** | **1.5** | **9.5** | **6** |

TABLE 11.3

| C-Compounds | IC50 (Antiviral) | CC50 (Toxicity) | SI (Selectivity Index) |
|---|---|---|---|
| Against HIV-IIIB | | | |
| C1 | 5 | 70 | 14 |
| | 36 | 70 | 2 |
| | 33 | 70 | 2 |
| | 35 | 60 | 2 |
| **Average** | **27** | **70** | **3** |
| Against HIV-1RF | | | |
| C1 | 7 | 60 | 8.5 |
| | - | - | 56 |
| | 16 | 56 | 3.5 |
| **Average** | **11.5** | **57** | **5** |
| Against chronically infected cells | | | |
| C1 | 16 | 30 | 2 |
| | 16 | 95 | 6 |
| **Average** | **16** | **63** | **4** |
| Against HIV-1IIIB | | | |
| C2 | 2 | 70 | 35 |
| C3 | 0.3 | 7 | 23 |
| C4 | 40 | 100 | 2.5 |
| | 30 | 70 | 2.3 |
| **Average** | **35** | **85** | **2.4** |
| C5 | 5 | 50 | 10 |
| C6 | 5 | 60 | 12 |
| C7 | 23 | 150 | 6 |
| | 5 | >200 | >10 |
| **Average** | **22** | **>175** | **8** |
| C8 | 10 | 60 | 5 |
| C9 | >200 | >200 | - |
| C-10 | >200 | >200 | - |
| C-11 | >200 | >200 | - |
| C-12 | >200 | >200 | - |

TABLE 11.4

| S-Compounds | IC50 (Antiviral) | CC50 (Toxicity) | SI (Selectivity Index) |
|---|---|---|---|
| Against HIV-1RF | | | |
| S1 | 20 | 100 | 5 |
| | 19 | 60 | 3 |
| **Average** | **20** | **80** | **4** |
| S2 | NR | | |
| S3 | NR | | |
| S4 | >200 | >200 | - |
| S5 | >200 | >200 | - |
| S6 | >200 | >200 | - |
| S7 | >200 | >200 | - |
| S8 | 40 | 100 | 2.5 |
| | 30 | 70 | 2 |
| **Average** | **35** | **75** | **2.4** |

TABLE 11.5

| E-Compounds | IC50 (Antiviral) | CC50 (Toxicity) | SI (Selectivity Index) |
|---|---|---|---|
| Against HIV-1RF | | | |
| E1 | 4 | 10 | 2.5 |
| E2 | 4 | 13 | 3 |
| E3 | 4 | 7 | 1.5 |
| E4 | 80 | >200 | 1.5 |
| E5 | 180 | >200 | 1 |
| E6 | 110 | >200 | 2 |
| E7 | >200 | >200 | - |
| E8 | 120 | >200 | 1.5 |
| E9 | ND | | |
| E10 | >200 | 90 | - |
| E11 | >200 | >200 | - |
| E12 | >200 | >200 | - |
| E13 | >200 | 80 | - |
| E14 | >200 | >200 | - |

While the invention has been described above in various specific details, it will be appreciated that numerous and various modifications may be made within the spirit and scope of the claims which follow. Thus, for example, the functional groups can be in various other positions, of which the above specifically recited are examples only.

## Claims

**1.** A compound for use as a pharmaceutical, the compound comprising an aromatic ring structure having at least one labile leaving moiety and at least one electrophilic moiety.

**2.** A compound as claimed in Claim 1 and having the general formula:

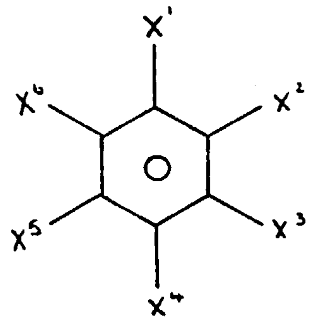

I

wherein one of $X^1$ to $X^6$ is a labile leaving moiety, one of the balance thereof is an electrophilic moiety and the remainder are the same or different and are hydrogen or a substituent.

**3.** A compound as claimed in Claim 2 wherein $X^1$ is a labile leaving moiety, one of $X^2$ to $X^6$ is an electrophilic moiety and the remainder are, each independently, hydrogen or a substituent, provided that when $X^2$ and $X^6$ are nitro groups, $X^4$ is neither a nitro group, a sulphonic acid group nor a sulphonate group or $X^1$ is not a labile group as defined below, namely a hydroxy group, an amino group, a sulfo group, a carboxy group, a methyloxy group, halogen or a hydrazyl group of the formula:

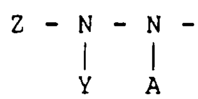

wherein A is hydrogen or an unpaired electron of the nitrogen atom, Y is hydrogen or an organic group and Z is an organic group, or Y and Z together with the adjacent nitrogen atom form a nitrogen-containing heterocycle.

**4.** A compound as claimed in Claim 2 wherein one of $X^1$ to $X^6$ is a labile leaving moiety, one of the balance thereof is an electrophilic moiety, and the remainder are the same or different and are hydrogen or an substituent with at least two thereof being other than nitro, at least one being a labile moiety and at least one being an electrophilic moiety.

**5.** A compound as claimed in any one of Claims 2 to 4, wherein at least one of $X^1$ to $X^6$ is an electrophilic moiety or labile moiety selected from the following:-

| | |
|---|---|
| electrophilic moieties | - $SO_3H$, $SO_3M$ (where M is a metal), halogen and $NO_2$ |
| labile moieties | - halogen, $SO_3H$, $SO_3M$ (where M is a metal), optionally substituted $NH_2$, COOH, optionally substituted $CONH_2$ and $COOR_3$ (where $R_3$ is a metal or alkyl). |

**6.** A compound as claimed in any preceding claim which has the general formula:

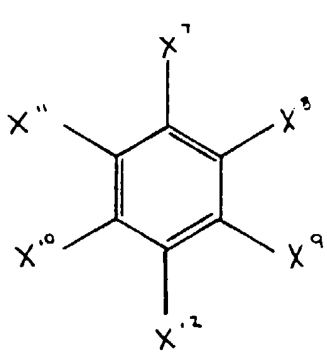

II

wherein:-

| | |
|---|---|
| $X^7$ | is $SO_3H$, $SO_3M$ (where M is a metal), halogen, COQ (where Q is hydroxy, amino or substituted amino, or the group $OR_3$ in which $R_3$ is a metal or alkyl), $NH_2$, substituted $NH_2$, $NO_2$ or OH; |
| $X^8$ | is hydrogen, halogen, glutathione or nitro; |
| $X^9$, $X^{10}$ and $X^{11}$ | are, each independently, hydrogen, halogen or nitro; and |
| $X^{12}$ | is hydrogen, nitro, optionally substituted amino, halogen, alkyl or glutathione. |

**7.** A compound as claimed in Claim 6 wherein:-

| | |
|---|---|
| $X^7$ | is $SO_3H$; |
| $X^8$ | is hydrogen, halogen or glutathione; |
| $X^9$ and $X^{10}$ | are, each independently, hydrogen, halogen or nitro; |
| $X^{11}$ | is hydrogen; and |
| $X^{12}$ | is hydrogen, nitro, optionally substituted amino, halogen, alkyl or glutathione. |

**8.** A compound as claimed in Claim 7 and as set forth by name below:-

8.1 4-chloro-3,5-dinitrobenzenesulphonic acid
8.2 4-chlorobenzenesulphonic acid
8.3 2,5-dichlorobenzenesulphonic acid
8.4 4-amino-3,5-dinitrobenzenesulphonic acid
8.5 3-nitro-4-methylbenzenesulphonic acid
8.6 2-chloro-3,5-dinitrobenzenesulphonic acid
8.7 2-glutathionyl-3,5-dinitrobenzenesulphonic acid
8.8 4-glutathionyl-3,5-dinitrobenzenesulphonic acid
8.9 3-nitro-4-methylbenzenesulphonic acid
8.10 3-nitro-4-chlorobenzenesulphonic acid
8.11 2,4-dinitrobenzenesulphonic acid
8.12 4-chloro-3,5-dinitrobenzene sulfonic acid
8.13 a salt of any of the acids listed as 8.1 and 8.12

**9.** A compound as claimed in Claim 6 wherein:-

| | |
|---|---|
| $X^7$ | is halogen; |
| $X^8$, $X^9$, $X^{10}$ and $X^{12}$ | are, each independently, hydrogen, halogen or nitro; and |
| $X^{11}$ | is hydrogen. |

**10.** A compound as claimed in Claim 9 and as set forth by name below:-

10.1 2,4-dinitrochlorobenzene
10.2 3,4-dinitrochlorobenzene
10.3 2,4-dinitrofluorobenzene
10.4 1,2-dichloro-4,5-dinitrobenzene
10.5 1,3-dichloro-4,5-dinitrobenzene
10.6 1,5-dichloro-2,3-dinitrobenzene

**11.** A compound as claimed in Claim 6 wherein:-

$X^7$ is $SO_3H$, $SO_3M$ (where M is a metal), $NH_2$ or substituted $NH_2$, halogen or hydroxy;

$X^8$ is nitro;

$X^9$ is hydrogen;

$X^{10}$ is hydrgen;

$X^{11}$ is nitro; and

$X^{12}$ is nitro.

**12.** A compound as claimed in Claim 11 nd as set forth by name below:-

12.1 2,4,6-trinitrophenol (picric acid),
12.2 2,4,6-trinitroaniline,
12.3 2,4,6-trinitrochlorobenzene.

**13.** A compound as claimed in Claim 6 wherein:-

$X^7$ is a group of formula-COQ in which Q is hydroxy, optionally substituted amino or has the formula $-OR_3$ in which $R_3$ is alkyl or metal;

$X^8$ is hydrogen or halogen;

$X^9$ and $X^{10}$ are, each independently, hydrogen, halogen or nitro;

$X^{11}$ is hydrogen; and

$X^{12}$ is hydrogen, nitro, optionally substituted amino or halogen.

**14.** A compound as claimed in Claim 13 and as set forth below by name:-

14.1 2-chloro-5-nitrobenzoic acid
14.2 2,4-dichloro-3,5-dinitrobenzoic acid or its alkyl ester
14.3 4-chloro-3,5-dinitrobenzoic acid or its alkyl ester
14.4 2,5-dichlorobenzoic acid
14.5 2,4-dinitrobenzoic acid
14.6 3,5-dinitrobenzoic acid
14.7 3-nitro-4-chloroanisole
14.8 4-amino-3,5-dinitrobenzamide
14.9 4-chloro-3,5-dinitrobenzamide
14.10 2,4-dichloro-3,5-dinitrobenzamide

**15.** A compound as claimed in Claim 6 wherein:

$X^7$ is optionally substituted amino; and

$R^8$ to $R^{12}$ are, each independently, hydrogen, halogen or nitro.

**16.** A compound as claimed in Claim 15 and as set forth below by name:-

16.1 2,6-dinitroaniline
16.2 2,4-dinitroaniline
16.3 3,5-dinitroaniline
16.4 2,4-dinitro-6-chloroaniline
16.5 2,6-dinitro-4-chloroaniline
16.6 2-chloro-4-nitroaniline

16.7 2,4-dinitro-5-fluoroaniline

**17.** A compound as claimed in any one of Claims 1 to 5 wherein a ring hydrogen provides a labile moiety, the compound having the general formula:

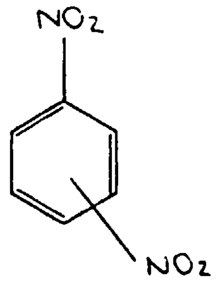

(VIII)

**18.** A compound as claimed in Claim 17 and as set forth by name below:-

18.1 1,2-dinitrobenzene
18.2 1,3-dinitrobenzene
18.3 1,4-dinitrobenzene

**19.** A compound as claimed in any one of Claims 2 to 5, wherein at least one of $X^1$ to $X^6$ is selected from:-

| | |
|---|---|
| labile moiety/moieties | - 1 or 2 halo groups and/or $NH_2$ or substituted $NH_2$ and/or COOH or substituted COOH and/or alkyl and/or $SO_3H/SO_3M$ |
| electrophilic moiety/moieties | - 1 or 2 nitro groups and/or $SO_3H/SO_3M$ and/or 1 or 2 halo groups |

**20.** A compound for use in the treatment or prevention of cancer, pre-cancer or disease caused by viral infection, which compound comprises an aromatic ring structure having at least one labile leaving moiety and at least one electrophilic moiety.

**21.** A compound for use in the treatment or prevention of cancer, pre-cancer or disease caused by viral infection, the compound being selected from the following classes of organic compounds:-

21.1 chlorodinitrobenzenesulphonic acid
21.2 chlorobenzenesulphonic acid
21.3 dichlorobenzenesulphonic acid
21.4 aminodinitrobenzenesulphonic acid
21.5 nitromethylbenzenesulphonic acid
21.6 glutathionyldinitrobenzenesulphonic acid
21.7 nitrochlorobenzenesulphonic acid
21.8 dinitrobenzenesulphonic acid
21.9 dinitrochlorobenzene
21.10 dinitrofluorobenzene
21.11 dichlorodinitrobenzene
21.12 trinitrophenol e.g. picric acid
21.13 trinitroaniline
21.14 trinitrochlorobenzene
21.15 trinitrobenzenesulphonic acid
21.16 chloronitrobenzoic acid
21.17 chlorodinitrobenzoic acid
21.18 dichlorobenzoic acid
21.19 dichloronitrobenzoic acid
21.20 dichlorodinitrobenzoic acid
21.21 dinitrobenzoic acid
21.22 nitrochloroanisole

21.23 aminodinitrobenzamide
21.24 dinitroaniline
21.25 dinitrochloroaniline
21.26 chloronitroaniline
21.27 dinitrofluoroaniline

**22.** A compound for use in the treatment or prevention of cancer, pre-cancer or disease caused by viral infection, the compound being a compound as set forth below by name:-

22.1 2,4,6-trinitrophenol
22.2 2,4-dichloro-3,5-dinitrobenzoic acid
22.3 4-chloro-3,5-dinitrobenzoic acid

**23.** A compound for use in the treatment or prevention of cancer or pre-cancer, the compound being a compound as set forth below by name:-

23.1 1,5-dichloro-2,3-dinitrobenzene
23.2 2-chloro-5-nitrobenzoic acid
23.3 4-chlorobenzenesulfonic acid
23.4 4-chloro-3,5-dinitrobenzene sulfonic acid

**24.** A compound for use in the treatment or prevention of disease caused by viral infection, the compound being a compound as set forth below by name:-

24.1 4-chloro-3,5-dinitrobenzamide
24.2 2,4-dichloro-3,5-dinitrobenzamide

**25.** A pharmaceutical composition, which composition comprises a compound according to any preceding claim and a pharmaceutically-acceptable diluent or carrier.

**26.** A composition as claimed in Claim 25, wherein the diluent or carrier is aqueous.

**27.** A composition as claimed in Claim 25 or Claim 26 which is in unit dosage form.

**28.** A composition as claimed in Claim 27 which is in the form of a tablet, capsule, powder, solution or suspension.

**29.** Use of a compound as claimed in any one of Claim 1 to 24 for the preparation of a medicament for the prophylaxis or therapy of cancer, pre-cancer or viral infection.

**30.** Use as claimed in Claim 29 wherein the compound is at a concentration and dose which enables an arylating mechanism to be brought into play.

**31.** A method of treating disease caused by viral infection, which method comprises administering an effective amount of a compound as claimed in any one of Claims 1 to 24 or a composition as claimed in any one of Claims 25 to 28.

**32.** A method of treating cancer or pre-cancer to reduce or eliminate cancerous growth, which method comprises administering an effective amount of a compound as claimed in any one of Claims 1 to 24 or a composition as claimed in any one of Claims 25 to 28.

**33.** A chloro- or nitro-benzenesulfonic acid compound, a chloro- or nitro-benzoic acid compound or chloro- or nitrobenzamide compound for use as a pharmaceutical.

FIG.1

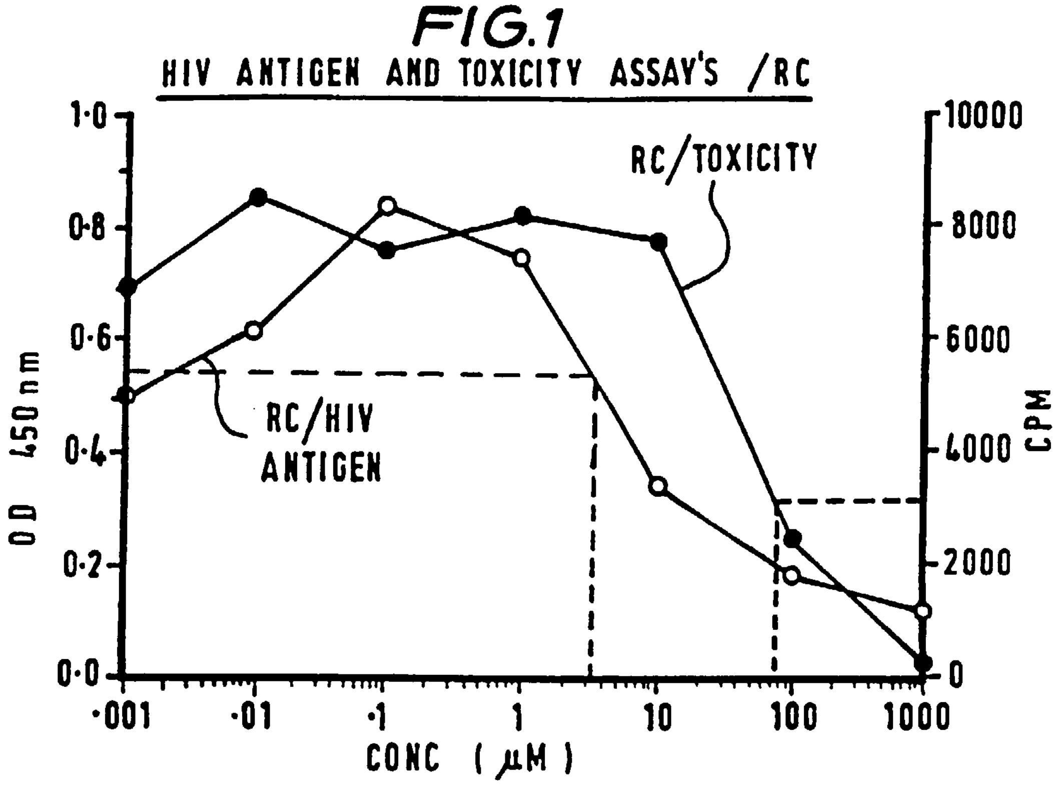
HIV ANTIGEN AND TOXICITY ASSAY'S /RC
RC/TOXICITY
RC/HIV ANTIGEN
OD 450nm
CPM
1·0
0·8
0·6
0·4
0·2
0·0
10000
8000
6000
4000
2000
0
·001
·01
·1
1
10
100
1000
CONC (μM)

FIG.2

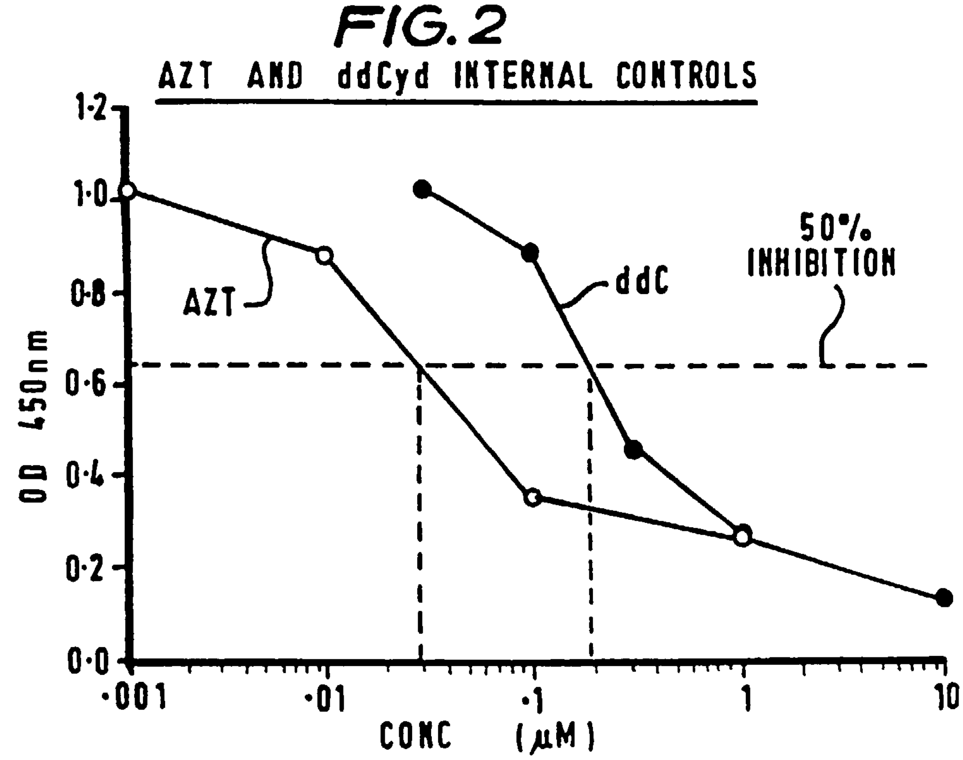
AZT AND ddCyd INTERNAL CONTROLS
50% INHIBITION
AZT
ddC
OD 450nm
1·2
1·0
0·8
0·6
0·4
0·2
0·0
·001
·01
·1
1
10
CONC (μM)

FIG.3

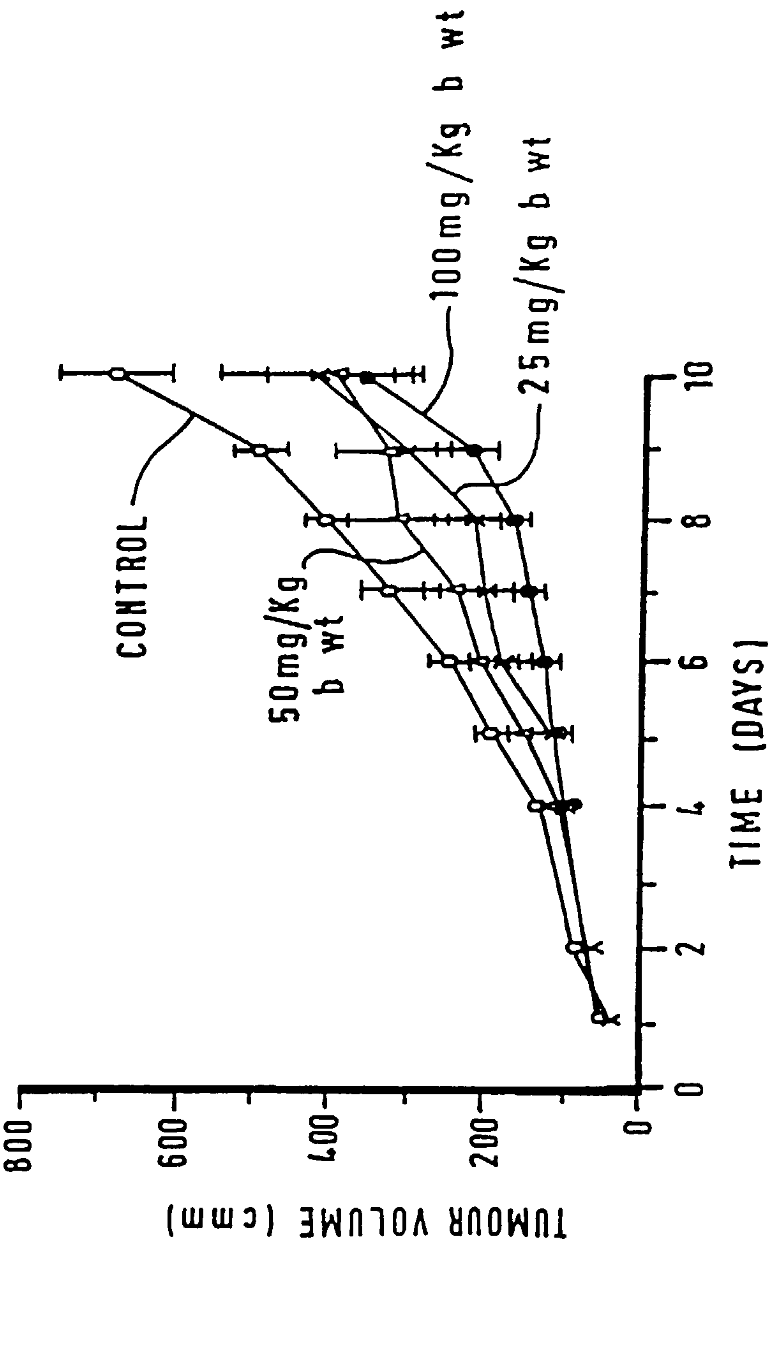
CONTROL
50mg/Kg b wt
100mg/Kg b wt
25mg/Kg b wt
TUMOUR VOLUME (c mm)
800
600
400
200
0
0
2
4
6
8
10
TIME (DAYS)

FIG. 4

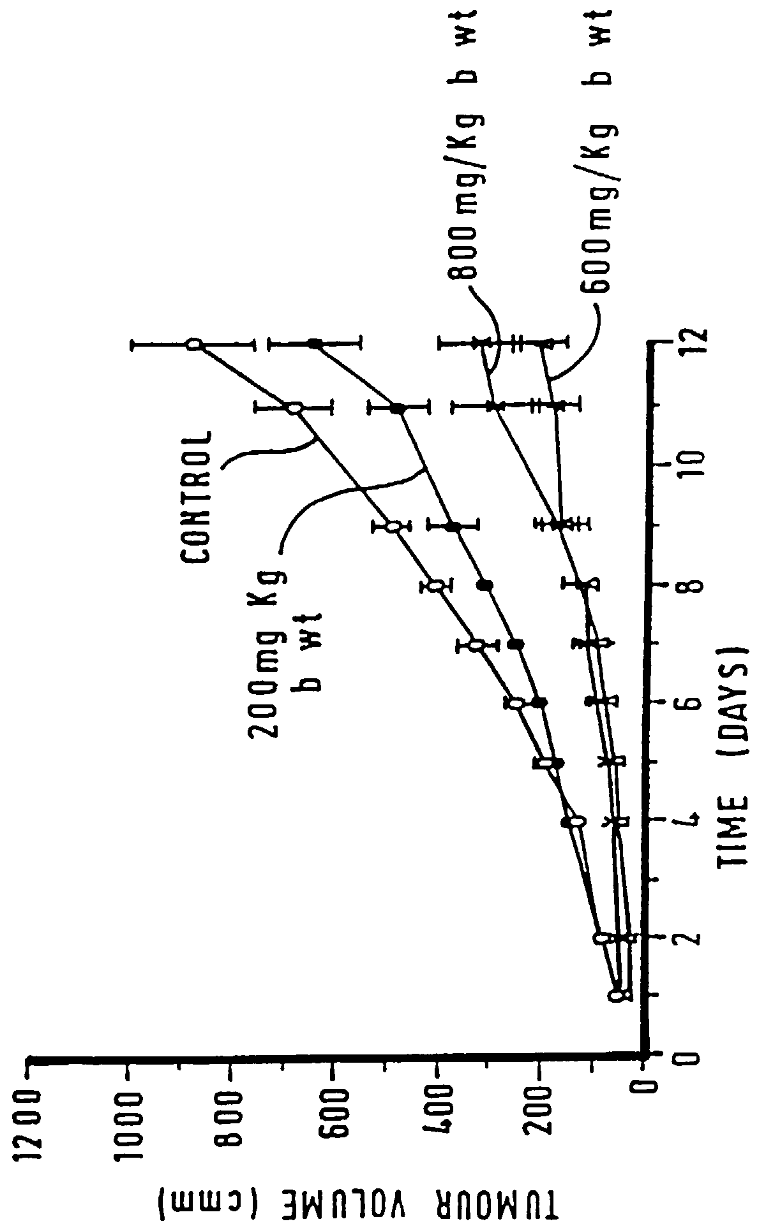
TUMOUR VOLUME (cmm)
1200
1000
800
600
400
200
0
0
2
4
6
8
10
12
TIME (DAYS)
CONTROL
200mg Kg b wt
800mg/Kg b wt
600mg/Kg b wt